# EUROPÄISCHE PATENTSCHRIFT

(11) **EP 0 809 663 B1**
(45) Veröffentlichungstag und Bekanntmachung des Hinweises auf die Patenterteilung: **29.07.1998**
(21) Anmeldenummer: 96903959.3
(22) Anmeldetag: 01.02.1996
(51) Int. Cl.: C08G 18/78

(54) **VERFAHREN ZUR HERSTELLUNG VON BIURETGRUPPEN-HALTIGEN POLYISOCYANATEN**
PROCESS FOR PRODUCING POLYISOCYANATES CONTAINING BIURET GROUPS
PROCEDE DE FABRICATION DE POLYISOCYANATES CONTENANT DES GROUPES BIURET

(30) Priorität: 15.02.1995 DE 19505035
(43) Veröffentlichungstag der Anmeldung: 03.12.1997
(73) Patentinhaber: BASF AKTIENGESELLSCHAFT, 67056 Ludwigshafen (DE)
(72) Erfinder: BRUCHMANN, Bernd, D-67069 Ludwigshafen (DE); WOLFF, Stefan, D-67117 Limburgerhof (DE); HEIDER, Wolfgang, D-67434 Neustadt (DE); JÄHME, Joachim, D-67240 Bobenheim-Roxheim (DE); LANGER, Werner, D-67061 Ludwigshafen (DE); RENZ, Hans, D-67149 Meckenheim (DE)
(86) Internationale Anmeldenummer: EP9600419
(87) Internationale Veröffentlichungsnummer: WO9625444

(56) Entgegenhaltungen:
- DE-A- 1 931 055
- DE-B- 1 227 004
- FR-A- 1 375 463
- FR-A- 1 475 617
- US-A- 3 976 622
- JOURNAL FÜR PRAKTISCHE CHEMIE CHEMIKER-ZEITUNG, Bd. 336, Nr. 3, 1994, LEIPZIG, DE, Seiten 185-200, XP000441642 LAAS ET AL: "ZUR SYNTHESE ALIPHATISCHER POLYISOCYANATE-LACKPOLYISOCYANATE MIT BIURET-, ISOCYANURAT- ODER URETDIONSTRUKTUR"

## Beschreibung

Die vorliegende Erfindung betrifft ein Verfahren zur Herstellung von Polyisocyanaten, die eine oder mehrere Biuretgruppen enthalten, durch Umsetzung von
a) einem aliphatischen oder cycloaliphatischen Polyisocyanat (Isocyanat a) mit
b) einem tertiären Alkohol oder einer Mischung aus Wasser und einem tertiären Alkohol (Biuretisierungsmittel b)
bei Reaktionstemperaturen von 100 bis 250°C.

Die Herstellung von biuretgruppen-haltigen Polyisocyanaten ist eine ausführlich beschriebene Reaktion (vgl. H.J. Laas et al., J. prakt. Chem. 336 (1994) 185-200).

Aus vielen Patenten ist beispielsweise bekannt, Wasser mit einem Überschuß an mehrwertigen Isocyanaten umzusetzen, wobei zunächst Harnstoffgruppen entstehen, die mit den Isocyanaten zu Biuretgruppen weiterreagieren (vgl. DE-A 1 101 394). Weil es schwierig ist, das Wasser und das Isocyanat homogen miteinander zu vermischen, werden dabei in der Praxis durch lokalen Wasserüberschuß immer mehr oder weniger große Anteile an unlöslichen polymeren harnstoffhaltigen Verbindungen erhalten, die sich im Reaktionsgefäß oder im Abgasraum abscheiden.

Die US-A 4 028 392 beschreibt ein Verfahren, bei dem man dieses Problem dadurch umgeht, daß man das Wasser in Form einer wäßrigen Lösung mit einem gegenüber Isocyanaten inerten Lösungsmittel einsetzt. Der Nachteil besteht jedoch darin, daß dabei das Lösungsmittel wieder durch Destillation vom Produkt abgetrennt werden muß.

Diese Probleme können durch das aus der DE-A 1 543 178 bekannte Verfahren überwunden werden, bei dem man anstelle des Wassers einen einwertigen tertiären Alkohol, z.B. tert.-Butanol, einsetzt. Der Alkohol reagiert bei Temperaturen von mindestens 70°C mit einem Überschuß an Isocyanat zu biuretgruppen-haltigen Polyisocyanaten, wobei als Nebenprodukte ein Olefin, z.B. Isobuten, und CO₂ gebildet werden, die sich leicht aus der Reaktionsmischung entfernen lassen.

Es wird vermutet, daß der Alkohol und das Isocyanat zunächst zu einem Urethan reagieren, das in ein Amin, CO₂ und ein Olefin zerfällt, und das Amin mit weiterem Isocyanat zu Harnstoffderivaten und dann zu biuretgruppen-haltigen Polyisocyanaten reagiert.

Diese Umsetzung wird bevorzugt in Gegenwart von Katalysatoren durchgeführt, wobei hierfür Säuren wie starke anorganische Lewis- und Brønstedt-Säuren (vgl. DE-A 1 543 178) und Salze aus stickstoffhaltigen Basen und anorganischen und/oder organischen Säuren (vgl. DE-A 1 931 055) empfohlen werden.

Biuretgruppen-haltige Polyisocyanate werden vor allem in der Lackindustrie als Härtungsmittel in Lacksystemen eingesetzt, die als Bindemittel im allgemeinen Polymere mit gegenüber Isocyanaten reaktiven Gruppen enthalten.

Damit die Lacksysteme nach dem Auftragen auf ein Substrat innerhalb kurzer Zeit zu Beschichtungen mit guten mechanischen Eigenschaften und einer hohen Widerstandsfähigkeit gegenüber Chemikalien aushärten, ist es erforderlich, daß die biuretgruppen-haltigen Polyisocyanate einen hohen Gehalt an NCO-Gruppen und eine hohe Reaktivität gegenüber den reaktiven Gruppen der Bindemittel aufweisen.

Weiterhin soll der Anteil an flüchtigen Isocyanaten auch nach längerer Lagerung gering sein, damit sich die biuretgruppen-haltigen Polyisocyanate, ohne daß hierzu besondere Schutzvorkehrungen erforderlich wären, gefahrlos verarbeiten lassen. Um mit ihnen Lacksysteme herstellen zu können, die gute Verlaufseigenschaften und einen niedrigen Lösungsmittelgehalt aufweisen, werden von der Lackindustrie Produkte verlangt, die gleichzeitig eine niedrige Viskosität aufweisen. Außerdem sollen die Produkte möglichst farblos sein.

Die nach den bekannten Verfahren aus tertiären Alkoholen und Isocyanaten hergestellten biuretgruppen-haltigen Polyisocyanate lassen jedoch noch zu wünschen übrig, da sie eine für viele Anwendungen zu dunkle Farbe aufweisen und insbesondere nach längerer Lagerung noch erhebliche Mengen an leicht flüchtigen monomeren Isocyanaten enthalten.

Der Erfindung liegt die Aufgabe zugrunde, ein wirtschaftliches Verfahren bereitzustellen, nach dem sich biuretgruppen-haltige Polyisocyanate herstellen lassen, die eine helle Farbe aufweisen und deren Gehalt an leichtflüchtigen Isocyanaten insbesondere nach längerer Lagerung niedrig ist.

Demgemäß wurde ein Verfahren zur Herstellung von Polyisocyanaten gefunden, die eine oder mehrere Biuretgruppen enthalten, durch Umsetzung von
a) einem aliphatischen oder cycloaliphatischen Isocyanat mit mehreren Isocyanatgruppen (Isocyanat a) mit
b) einem tertiären Alkohol oder einer Mischung aus Wasser und einem tertiären Alkohol (Biuretisierungsmittel b)
bei Reaktionstemperaturen von 100 bis 250°C, dadurch gekennzeichnet, daß man die Umsetzung in Gegenwart
c) eines Stabilisators (c), bei dem es sich um katalytische Mengen Harnstoff, Ammoniak, Biuret, ein Harnstoffderivat der Formel I 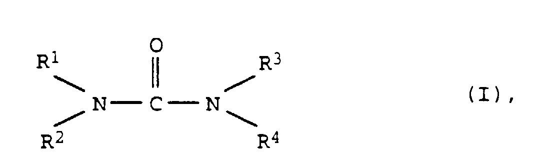 in der R¹, R², R³ und R⁴ Wasserstoff, C₁- bis C₁₀-Alkyl oder C₅- bis C₁₀-Aryl bedeuten, oder
   um ein Carbonsäureamid der Formel II 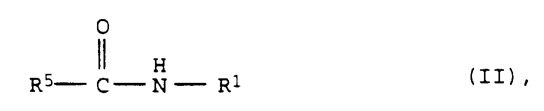 in der R⁵ eine C₁- bis C₁₂-Alkylgruppe bedeutet, in der gegebenenfalls 1, 2 oder 3 Wasserstoffatome durch einen Rest 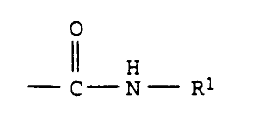 ersetzt sind, handelt,
durchführt.

Als Ausgangsstoffe für das erfindungsgemäße Verfahren kommen als Isocyanate (a) mehrwertige Isocyanate, insbesondere aliphatische und cycloaliphatische Di- und Tri-Isocyanate, die 4 bis 30 C-Atomen enthalten, in Betracht. Insbesondere zu nennen sind Diisocyanate X(NCO)₂, wobei X für einen aliphatischen Kohlenwasserstoffrest mit 4 bis 12 Kohlenstoffatomen oder einen cycloaliphatischen Kohlenwasserstoffrest mit 6 bis 15 Kohlenstoffatomen steht. Von besonderer Bedeutung sind hier die kommerziell erhältlichen Ausgangsverbindungen, die industriell nach den z.B. aus der DE-PS 20 05 309 und DE-OS 2 404 773 beschriebenen Verfahren durch Phosgenierung von Diaminen und nach den in der EP-B-0 126 299 (US-A-4 596 678), EP-B-0 126 300 (US-A-4 596 679), EP-A-0 355 443 (US-A-5 087 739) sowie der EP-A-0 568 782 beschriebenen phosgenfreien Verfahren (Spaltung von Biurethanen) hergestellt werden.

Dies sind insbesondere 1,6-Diisocyanatohexan (HDI), 1-Isocyanato-3-isocyanatomethyl-3,5,5-trimethylcyclohexan (IPDI) sowie Bis(4-isocyanatocyclohexyl)methan.

In der Praxis von weniger großer Bedeutung, jedoch prinzipiell ebenfalls geeignet, sind als Ausgangsverbindungen Isocyanate mit 3 und mehr Isocyanatgruppen, z.B. solchen, die zusätzlich Allophanat- oder Isocyanuratgruppen enthalten. Beispiele hierfür sind die entsprechenden Derivate des HDI, die durch Trimerisierung von HDI hergestellt werden (vgl. Kunststoff Handbuch, Band 7, S. 94 bis 96, 3. Auflage, 1993, Carl Hanser Verlag).

Als Biuretisierungsmittel (b) kommen insbesondere die in der DE-OS 1 543 178 genannten tertiären Alkohole in Betracht, also vor allem einwertige Alkohole mit 4 bis 20 Kohlenstoffatomen, z.B. 2-Methyl-2-butanol, 2-Methyl-2-pentanol, 3-Methyl-3-pentanol, 3-Äthyl-3-pentanol, 3-Äthyl-3-nonanol, 3-Methyl-1-butyn-3-ol, 3-Methyl-1-pentyn-3-ol, 3,5-Dimethyl-1-hexyn-3-ol, 1-Methylcyclopentanol, 1-Methylcyclohexanol, 1-Äthylcyclohexanol, 1,1-Diphenyläthanol, 1,1,2-Triphenyläthanol und vor allem tert.-Butylalkohol. Selbstverständlich sind auch Gemische dieser Alkohole geeignet.

Zur Biuretisierung der Isocyanate (a) kann neben den tertiären Alkoholen auch Wasser in Form einer wäßrigen Lösung mit den tertiären Alkoholen eingesetzt werden. Dabei kommen vor allem Lösungen aus dem tertiären Alkohol und Wasser in Betracht, die bis zu 80, bevorzugt bis zu 40 mol-% Wasser, bezogen auf die Summe der Mischungskomponenten, enthalten, weil bei diesen Mischungsverhältnissen Wasser homogen eingebracht wird und bei der Umsetzung mit den Isocyanaten (a) noch keine oligomeren und polymeren Harnstoffderivate, die aus der Reaktionsmischung ausfallen, gebildet werden.

Erfindungsgemäß erfolgt die Umsetzung des Isocyanats (a) mit dem Biuretisierungsmittel (b) in Gegenwart von katalytischen Mengen eines Stabilisators (c).

Als Stabilisatoren (c) eignen sich Harnstoff, Ammoniak, Biuret, ein Harnstoffderivat der Formel I 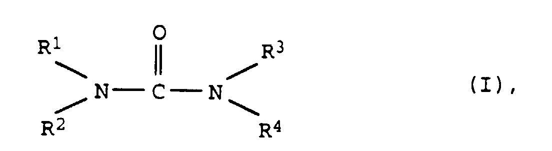 in der R¹, R², R³ und R⁴ Wasserstoff, C₁- bis C₁₀-Alkyl, bevorzugt Methyl oder Ethyl, oder C₅- bis C₁₀-Aryl, bevorzugt Phenyl oder Benzyl, bedeuten oder
ein Carbonsäureamid der Formel II 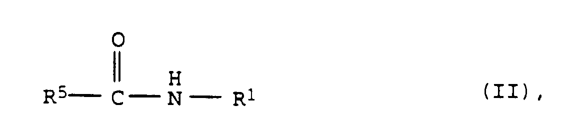 in der R⁵ eine C₁- bis C₁₂-Alkylgruppe, bevorzugt eine C₁- bis C₆-Alkylgruppe bedeutet, in der gegebenenfalls 1, 2 oder 3 Wasserstoffatome durch einen Rest 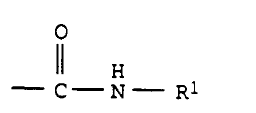 ersetzt sind,
durchführt.

Beispiele für geeignete Harnstoffderivate sind N-Methylharnstoff, N,N-Dimethylharnstoff, N,N'-Dimethylharnstoff, N-Ethylharnstoff, N,N-Diethylharnstoff, N,N'-Diethylharnstoff, Ethylenharnstoff sowie N-Phenylharnstoff.

Geeignete Carbonsäureamide der Formel II sind Formamid, N-Methylformamid, Acetamid, Malonsäurediamid sowie Bernsteinsäurediamid.

Die Stabilisatoren (c) werden bevorzugt in Mengen von 0,01 bis 2,0 mol-%, besonders bevorzugt in Mengen von 0,05 bis 1 mol-%, bezogen auf die Isocyanatgruppen im Isocyanat (a), eingesetzt.

Nach dem erfindungsgemäßen Verfahren kann die Herstellung des biuretgruppen-haltigen Polyisocyanats sowohl kontinuierlich als auch diskontinuierlich erfolgen.

Für die kontinuierliche Herstellung eignet sich z.B. eine aus mehreren Einzelreaktoren bestehende kontinuierlich durchflossene Reaktorkaskade.

Die diskontinuierliche Herstellung kann z.B. in einem Rührreaktor vorgenommen werden.

Üblicherweise wird das Isocyanat (a) vorgelegt und das Biuretisierungsmittel (b), in dem günstigerweise der Stabilisator (c) bereits gelöst ist, zudosiert.

Die Umsetzung wird bevorzugt in Substanz durchgeführt, jedoch kann zur Erniedrigung der Viskosität auch ein gegenüber Isocyanatgruppen inertes Lösungsmittel mitverwendet werden. Geeignete Lösungsmittel sind in der DE-OS 1 543 178 genannt, Dioxan, Tetrahydrofuran, Triethylenglykoldiacetat, Toluol, Benzol, Chlorbenzol, o-Dichlorbenzol, Butylacetat, Ethylenglykol-monoethylether-acetat und Methylenchlorid.

Die Umsetzung erfolgt im allgemeinen unter Normaldruck, höhere Drücke von 1 bis 10 bar empfehlen sich beispielsweise bei dem Einsatz von unterhalb der bevorzugten Reaktionstemperaturen siedenden Isocyanaten (a) oder Lösungsmitteln.

Die Reaktionszeiten betragen bei den bevorzugten Reaktionstemperaturen im allgemeinen 2 bis 5 h. Die Reaktionszeit wird dabei günstigerweise so gewählt, daß am Ende der theoretischen NCO-Wert erreicht ist. Der theoretische NCO-Wert ist der NCO-Wert, den die Reaktionsmischung aufweist, wenn die gesamte eingesetzte Menge an Biuretisierungsmittel die theoretisch zu erwartende Menge an Biuretgruppen gebildet hat.

In Folge der Umsetzung einer Isocyanatgruppe mit einem Wasser- oder tertiären Alkoholmolekül entsteht bekanntlich eine Aminogruppe, die mit zwei weiteren Isocyanatgruppen unter Bildung einer Biuretgruppe abreagiert. Da als Ausgangsprodukte mehrwertige Isocyanate eingesetzt werden, erfolgt das Anwachsen der biuretgruppen-haltigen Polyisocyanate deshalb nach der Kinetik der Vernetzungsreaktionen (vgl. B. Vollmert, Grundriß der Makromolekularen Chemie, Band II, S. 247 bis 260, Vollmert-Verlag, Karlsruhe, 1988), wobei jede Biuretgruppe einen Verzweigungspunkt bildet. Um die Bildung von größeren verzweigtkettigen Assoziaten mit mehreren Verzweigungspunkten oder gar Gelbildung zu vermeiden, empfiehlt es sich im allgemeinen, 0,5 bis 20 mol-%, bevorzugt 2 bis 10 mol-% Biuretisierungsmittel, bezogen auf die Isocyanatgruppen im Isocyanat (a), einzusetzen.

Unter diesen Bedingungen reagieren die Isocyanate (a) mit den Biuretisierungsmitteln vorwiegend zu Mischungen von biuretgruppen-haltigen Polyisocyanaten, die als Hauptkomponente solche biuretgruppen-haltige Polyisocyanate enthalten, welche aus drei vom Isocyanat (a) abgeleiteten Einheiten mit nur einer Biuretgruppe aufgebaut sind.

Im übrigen ist es durch einfache Vorversuche oder Berechnung möglich, die stöchiometrischen Verhältnisse zu ermitteln, bei denen Mischungen von biuretgruppen-haltigen Polyisocyanaten mit dem gewünschten mittleren Polymerisationsgrad gebildet werden.

Im allgemeinen wird es, um Produkte, die bei der Verarbeitung keine gefährlichen Mengen an Isocyanaten freisetzen, zu erhalten, erforderlich sein, den größten Teil der unumgesetzten Isocyanate (a) von den gebildeten biuretgruppen-haltigen Polyisocyanaten abzutrennen. Meistens werden Produkte gewünscht, deren Gehalt an den monomeren Isocyanaten (a) weniger als 1, bevorzugt weniger als 0,5 Gew.-%, bezogen auf die biuretgruppen-haltigen Polyisocyanate, beträgt. Die Abtrennung der Isocyanate (a) nimmt man günstigerweise bei vermindertem Druck bei Temperaturen vor, die zwischen 50°C und der bei der Umsetzung gewählten Reaktionstemperatur liegen, indem man sie beispielsweise abdestilliert.

In der Lackindustrie werden vor allem biuretgruppen-haltige Polyisocyanate, die weitgehend frei von Lösungsmitteln und den als Ausgangsstoffen eingesetzten Isocyanaten (a) sind und eine Viskosität von 2.000 bis 15.000, bevorzugt von 2.500 bis 10.000 mPa·s (gemessen bei einer Temperatur von 23°C und einem Schergefälle von 100 s⁻¹) aufweisen, gewünscht.

Produkte mit diesen Viskositäten werden im allgemeinen erhalten, wenn die Stöchiometrie der Ausgangsprodukte, der Isocyanate (a) und der Biuretisierungsmittel (b), wie empfohlen gewählt wird.

Die nach diesem Verfahren erhaltenen Produkte zeichnen sich insbesondere dadurch aus, daß sie bei vergleichsweise niedriger Viskosität und niedrigem Gehalt an niedermolekularen, flüchtigen Isocyanaten, wie den als Ausgangsprodukten eingesetzten Isocyanaten (a), einen hohen NCO-Gehalt und eine hohe Reaktivität gegenüber in Lacken eingesetzten Bindemitteln mit gegenüber Isocyanaten reaktiven Gruppen, z.B. hydroxylgruppen-haltigen Polyacrylaten aufweisen. Besonders vorteilhaft ist, daß sich der Gehalt an flüchtigen Isocyanaten auch bei längerer Lagerung der Produkte nicht erhöht und die Produkte weitgehend farblos sind.

Die nach dem erfindungsgemäßen Verfahren erhaltenen Produkte eignen sich insbesondere als Härter in der Lackindustrie. Die Verarbeitung dieser Härter zu Lacken und hieraus hergestellten Beschichtungen ist allgemein bekannt.

### Beispiele

### Allgemeine Herstellungsvorschrift für die biuretgruppen-haltigen Polyisocyanaten (a)

In einem 1 l-Rührreaktor wurden unter Stickstoffbedeckung 504 g (3 mol) 1,6-Hexamethylendiisocyanat (HDI) vorgelegt und auf die in den nachstehenden Tabellen angegebene Reaktionstemperatur aufgeheizt. Anschließend wurden 14 mol-%, bezogen auf das HDI, Biuretisierungsmittel (b) und darin gelost 0,2 mol-%, bezogen auf das HDI, des Stabilisators (c) bzw. des sauren Katalysators innerhalb von 2 min zugegeben und die Reaktionsmischung 3 h gerührt. Danach wurde die Reaktionsmischung an einem Dünnschichtverdampfer bei 165°C und 2,5 mbar destilliert.

Abweichend von den oben gemachten Angaben betrug die eingesetzte Harnstoffmenge bei
Beispiel 11 0,4 mol-%,
Beispiel 12 0,6 mol-% und
Beispiel 13 1,0 mol-%,
bezogen auf die HDI-Menge.

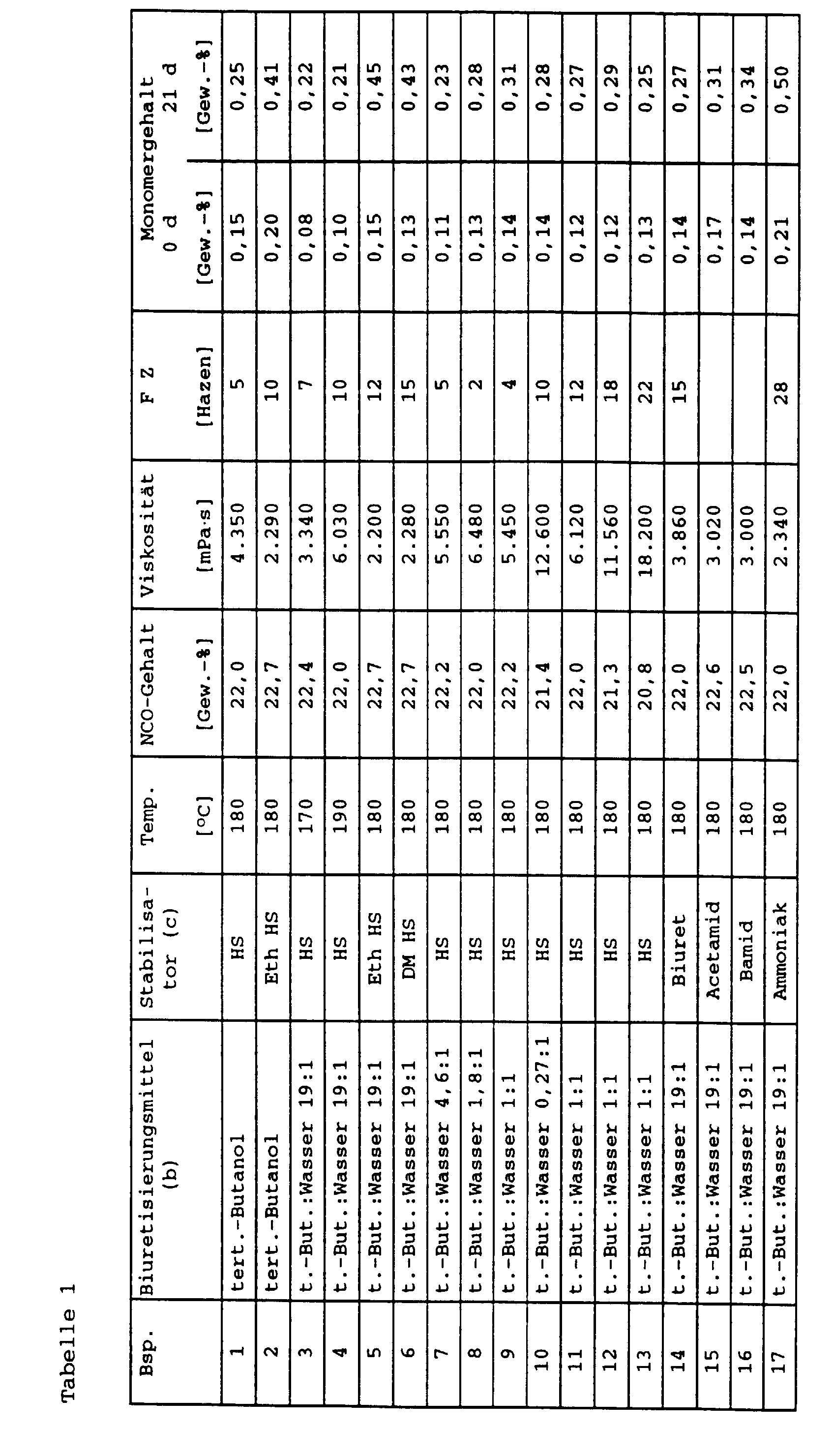

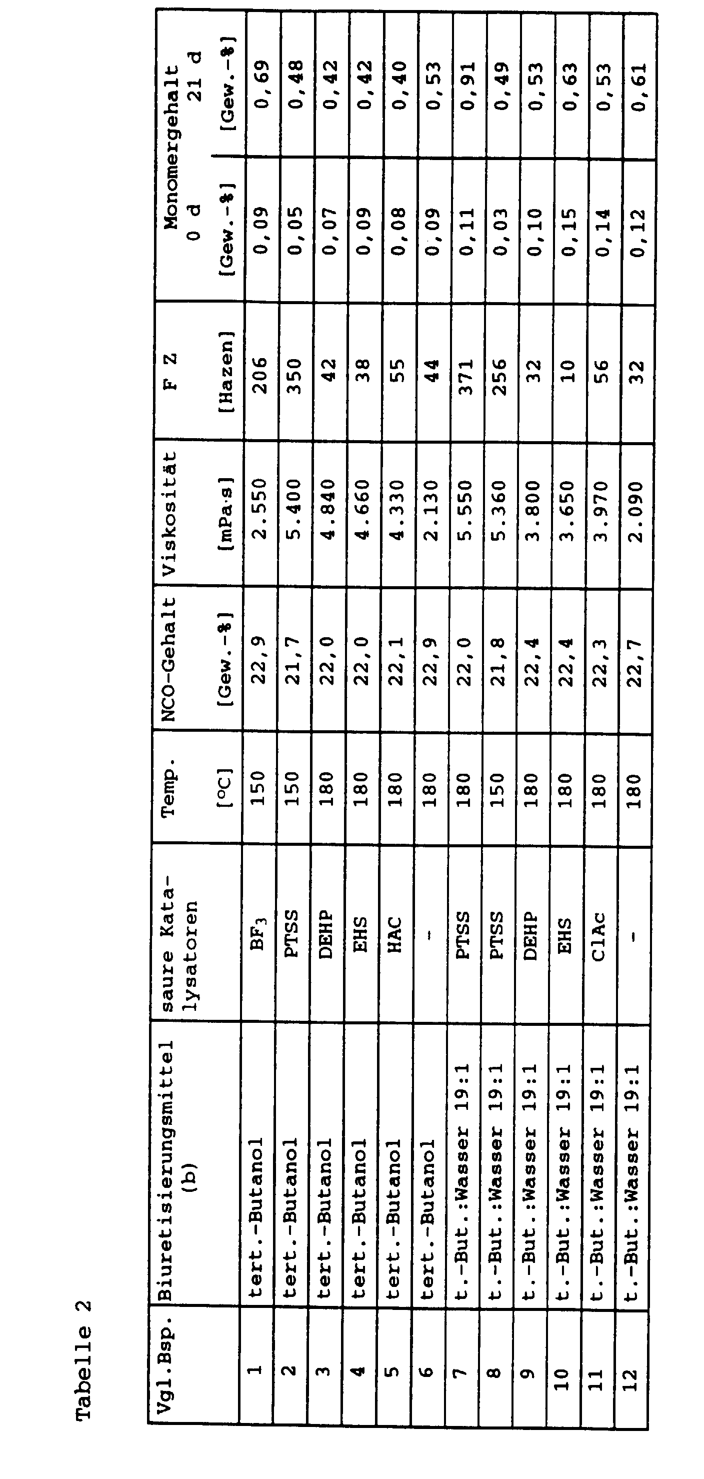

### Erläuterungen zu Tabelle 1 und 2

### Einsatzstoffe

Als Biuretisierungsmittel wurden tert.-Butanol (t.-But.) und Mischungen aus tert.-Butanol und Wasser eingesetzt. Angegeben ist jeweils das Molverhältnis der Mischungskomponenten
- HS =: Harnstoff
- Eth HS =: Ethylenharnstoff
- DMHS =: N,N'-Dimethylharnstoff
- BF₃ =: Bortrifluorid als Dihydrat
- PTSS =: p-Toluolsulfonsäure
- DEHP =: Di-(2-ethylhexyl)phosphat
- EHS =: 2-Ethylhexansäure
- HAc =: Essigsäure
- Bamid =: Bernsteinsäurediamid
- ClAc =: Chloressigsäure
- Ammoniak =: Ammoniak als 25 gew.-%ige wäßrige Lösung

NCO-Gehalt:
Der NCO-Gehalt ist in Gew.-% angegeben und wurde nach DIN 53 185 gemessen.

Viskosität:
Die Angaben zur Viskosität beziehen sich auf Messungen bei 23°C und einem Schergefälle von 100 s⁻¹.

Farbzahl (FZ):
Die Farbzahl wurde nach DIN ISO 6271 ermittelt und ist in Hazen angegeben.

Monomergehalt:
Der Monomergehalt gibt die Menge an monomerem Isocyanat in Gew.-% an, die sich unmittelbar nach der Herstellung (O d) bzw. nach 21-tägiger Lagerung bei 50°C (21 d) in dem jeweiligen biuretgruppen-haltigen Polyisocyanat befand.

Der Monomergehalt wurde nach DIN 55 956 gemessen.

## Patentansprüche

1. Verfahren zur Herstellung von Polyisocyanaten, die eine oder mehrere Biuretgruppen enthalten, durch Umsetzung von
a) einem aliphatischen oder cycloaliphatischen Isocyanat mit mehreren Isocyanatgruppen (Isocyanat a) mit
b) einem tertiären Alkohol oder einer Mischung aus Wasser und einem tertiären Alkohol (Biuretisierungsmittel b)
bei Reaktionstemperaturen von 100 bis 250°C, dadurch gekennzeichnet, daß man die Umsetzung in Gegenwart
c) eines Stabilisators (c), bei dem es sich um katalytische Mengen Harnstoff, Ammoniak, Biuret, ein Harnstoffderivat der Formel I 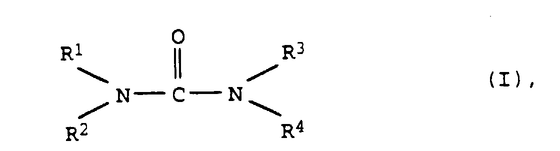 handelt, in der R¹, R², R³ und R⁴ Wasserstoff, C₁- bis C₁₀-Alkyl oder C₅- bis C₁₀-Aryl bedeuten, oder
um ein Carbonsäureamid der Formel II 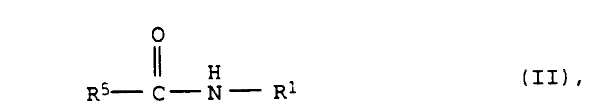 in der R⁵ eine C₁- bis C₁₂-Alkylgruppe bedeutet, in der gegebenenfalls 1, 2 oder 3 Wasserstoffatome durch einen Rest 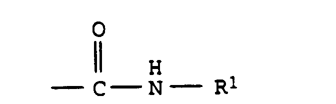 ersetzt sind,
durchführt.

2. Verfahren nach Anspruch 1, dadurch gekennzeichnet, daß man als Isocyanat (a) ein C₄- bis C₂₀- Di- oder Triisocyanat einsetzt.

3. Verfahren nach Anspruch 1 oder 2, dadurch gekennzeichnet, daß man als Isocyanat (a) Hexamethylen-1,6-diisocyanat einsetzt.

4. Verfahren nach den Ansprüchen 1 bis 3, dadurch gekennzeichnet, daß man als Biuretisierungsmittel (b) einen tertiären Alkohol oder Mischungen aus einem tertiären Alkohol und Wasser, die bis zu 80 mol-% Wasser, bezogen auf die Summe der Mischungskomponenten, enthalten, einsetzt.

5. Verfahren nach den Ansprüchen 1 bis 4, dadurch gekennzeichnet, daß man als tertiären Alkohol tert.-Butanol einsetzt.

6. Verfahren nach den Ansprüchen 1 bis 5, dadurch gekennzeichnet, daß man 0,5 bis 20 mol-% Biuretisierungsmittel (b), bezogen auf die Isocyanatgruppen im Isocyanat (a), einsetzt.

7. Verfahren nach den Ansprüchen 1 bis 6, dadurch gekennzeichnet, daß man 0,01 bis 2,0 mol-% eines Stabilisators (c), bezogen auf die Isocyanatgruppen im Isocyanat (a), einsetzt.

8. Verfahren nach den Ansprüchen 1 bis 7, dadurch gekennzeichnet, daß man die Umsetzung bei einer Reaktionstemperatur von 140 bis 220°C durchführt.

9. Verfahren nach den Ansprüchen 1 bis 7, dadurch gekennzeichnet, daß man im Anschluß an die Herstellung der biuretgruppen-haltigen Polyisocyanate nicht umgesetztes Isocyanat (a) von den biuretgruppen-haltigen Polyisocyanaten bis auf einen Gehalt von weniger als 0,5 Gew.-%, bezogen auf die biuretgruppen-haltigen Polyisocyanate, abtrennt.

## Claims

1. A process for the preparation of a polyisocyanate which contains one or more biuret groups by reacting
a) an aliphatic or cycloaliphatic isocyanate containing two or more isocyanate groups (isocyanate a) with
b) a tertiary alcohol or a mixture of water and a tertiary alcohol (biuretizing agent b)
at from 100 to 250°C, which comprises carrying out the reaction in the presence
c) of a stabilizer (c) which constitutes a catalytic amount of urea, ammonia, biuret, a urea derivative of the formula I 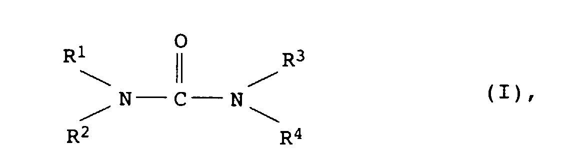 in which R¹, R², R³ and R⁴ are hydrogen, C₁ to C₁₀ alkyl or C₅ to C₁₀ aryl, or
a carboxamide of the formula II 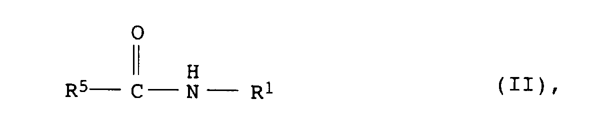 in which R⁵ is C₁ to C₁₂ alkyl which is unsubstituted or in which 1, 2 or 3 hydrogen atoms are replaced by a radical

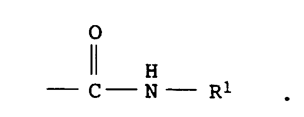

2. A process as claimed in claim 1, wherein the isocyanate (a) is a C₄ to C₂₀ diisocyanate or triisocyanate.

3. A process as claimed in claim 1 or 2, wherein the isocyanate (a) is hexamethylene-1,6-diisocyanate.

4. A process as claimed in any of claims 1 to 3, wherein the biuretizing agent (b) is a tertiary alcohol or a mixture of a tertiary alcohol and water including up to 80 mol% of water, based on the sum of the components of the mixture.

5. A process as claimed in any of claims 1 to 4, wherein the tertiary alcohol is tert-butanol.

6. A process as claimed in any of claims 1 to 5, wherein from 0.5 to 20 mol% of biuretizing agent (b) are employed, based on the isocyanate groups in (a).

7. A process as claimed in any of claims 1 to 6, wherein from 0.01 to 2.0 mol% of a stabilizer (c) are employed, based on the isocyanate groups in (a).

8. A process as claimed in any of claims 1 to 7, wherein the reaction is carried out at from 140 to 220°C.

9. A process as claimed in any of claims 1 to 7, wherein the polyisocyanate containing biuret groups is prepared and then unreacted isocyanate (a) is removed from it down to a content of less than 0.5% by weight, based on the polyisocyanate which contains biuret groups.

## Revendications

1. Procédé de préparation de polyisocyanates contenant un ou plusieurs groupements biuret, par réaction de
a) un isocyanate aliphatique ou cycloaliphatique ayant plusieurs groupements isocyanate (isocyanate a) avec
b) un alcool tertiaire ou un mélange d'eau et d'un alcool tertiaire (milieu de biurétisation b)
à des températures réactionnelles de 100-250°C, caractérisé en ce que l'on mène la réaction en présence
c) d'un agent stabilisant (c) constitué par des quantités catalytiques d'urée, d'ammoniaque, de biuret, d'un dérivé de l'urée de formule I 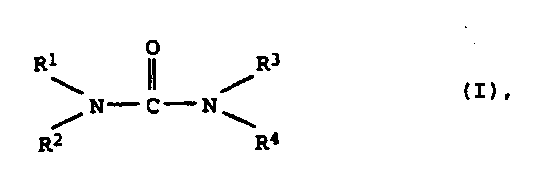 dans laquelle R¹, R², R³ et R⁴ représentent des atomes d'hydrogène, des groupements alkyle en C₁-C₁₀ ou aryle en C₅-C₁₀, ou constitué par un amide d'acide carboxylique de formule II 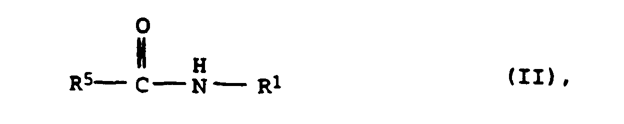 dans laquelle R⁵ représente un groupement alkyle en C₁-C₁₂ dans lequel 1, 2 ou 3 atomes d'hydrogène sont éventuellement remplacés par un reste

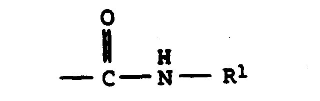

2. Procédé selon la revendication 1, caractérisé en ce que l'on utilise en tant qu'isocyanate (a) un di- ou triisocyanate en C₄-C₂₀.

3. Procédé selon la revendication 1 ou 2, caractérisé en ce que l'on utilise en tant qu'isocyanate (a) de l'hexaméthylène-1,6-diisocyanate.

4. Procédé selon l'une quelconque des revendications 1 à 3, caractérisé en ce que l'on utilise en tant que milieu de biurétisation (b) un alcool tertiaire ou des mélanges d'un alcool tertiaire et d'eau contenant jusqu'à 80% en moles d'eau, par rapport à la somme des constituants du mélange.

5. Procédé selon l'une quelconque des revendications 1 à 4, caractérisé en ce que l'on utilise du tert.-butanol en tant qu'alcool tertiaire.

6. Procédé selon l'une quelconque des revendications 1 à 5, caractérisé en ce que l'on utilise 0,5-20% en moles de milieu de biurétisation (b), par rapport aux groupements isocyanate présents dans l'isocyanate (a).

7. Procédé selon l'une quelconque des revendications 1 à 6, caractérisé en ce que l'on utilise 0,01-2,0% en moles d'un agent stabilisant (c), par rapport aux groupements isocyanate présents dans l'isocyanate (a).

8. Procédé selon l'une quelconque des revendications 1 à 7, caractérisé en ce que l'on mène la réaction à une température réactionnelle de 140-220°C.

9. Procédé selon l'une quelconque des revendications 1 à 7, caractérisé en ce que, à la suite de la préparation du polyisocyanate contenant des groupements biuret, on sépare l'isocyanate (a) n'ayant pas réagi du polyisocyanate contenant des groupements biuret, jusqu'à atteindre une teneur inférieure à 0,5% en poids, par rapport au polyisocyanate contenant des groupements biuret.
